# FASCICULE DE BREVET EUROPEEN

(11) **EP 1 750 770 B1**
(45) Date de publication et mention de la délivrance du brevet: **21.12.2011**
(21) Numéro de dépôt: 05717541.6
(22) Date de dépôt: 03.02.2005
(51) Int. Cl.: A61K 47/48, A61K 31/728, A61K 31/60, A61K 31/737

(54) **UTILISATION DE COMPOSES ORGANO-SILICIES POUR CONTRAINDRE DES TISSUS CONJONCTIFS LESES**
VERWENDUNG VON ORGANISCHEN SILIKONVERBINDUNGEN ZUM EINZWÄNGEN VON BESCHÄDIGTEM BINDEGEWEBE
USE OF ORGANO-SILICON COMPOUNDS FOR CONSTRAINING CONNECTIVE DAMAGED TISSUES

(30) Priorité: 05.02.2004 FR 0401095
(43) Date de publication de la demande: 14.02.2007
(73) Titulaire: Exsymol, 98000 Monaco (MC)
(72) Inventeur: SEGUIN, Marie-Christine, MC-98000 Monaco (MC); COURBEBAISSE, Yann, F-35760 Saint Gregoire (FR)
(74) Mandataire: Hubert, Philippe
(86) Numéro de dépôt international: PCT/FR2005/000229
(87) Numéro de publication internationale: WO 2005/082421

(56) Documents cités:
- EP-A- 0 842 938
- DE-A- 3 511 135
- FR-A- 2 158 068
- FR-A- 2 611 496
- FR-A- 2 781 675
- FR-M- 5 201
- US-A- 4 985 405
- DATABASE CAPLUS CHEMICAL ABSTRACTS SERVICE, COLUMBUS, OHIO, US; 4 août 1987 (1987-08-04), KUBO T.: "Colloidal silanol-containing deodorants for air purification" XP002100416 & JP 01 037955 A (KUBO GIJUTSU JIMUSHO:KK) 8 février 1989 (1989-02-08)
- PATENT ABSTRACTS OF JAPAN vol. 2003, no. 11, 5 novembre 2003 (2003-11-05) & JP 2003 183149 A (NOEVIR CO LTD), 3 juillet 2003 (2003-07-03)

## Description

La présente invention concerne l'utilisation de composés organo-siliciés pour contraindre l'organisation des fibres protéiniques et glycoprotéiniques de la matrice extracellulaire du derme lésé.

L'invention trouve application notamment dans le domaine des dispositifs médicaux.

A l'heure actuelle, il existe un besoin important de mettre au point des produits permettant d'améliorer une condition pathologique par la seule intervention d'une action mécanique, c'est-à-dire sans action pharmacologique directe.

Ce type de mode d'action s'avère particulièrement intéressant dans la mesure où l'on recherche des modes de traitement aussi peu agressifs que possible pour l'organisme. De tels traitements permettent, en outre, de satisfaire à la Directive communautaire 93/42/CEE concernant les dispositifs médicaux. Selon cette Directive, un dispositif médical est notamment défini par "une matière, utilisée seule ou en association, destinée à être utilisée chez l'homme à des fins de modification d'un processus physiologique mais dont l'action principale n'est pas obtenue par des moyens pharmacologiques ou immunologiques, ni par métabolisme".

Il existe diverses techniques de correction des dégradations ou altérations de tissus conjonctifs. Pour la peau par exemple sont proposés de nombreux produits communément appelés "de comblement" puisqu'ils agissent par un effet de "remplissage" du volume perdu cutané. Injectables, d'origine synthétique ou naturelle, ces produits sont le plus souvent à base de collagène, d'acide hyaluronique (Klein A.W., Facial Plast. Surg. Clin. North Am. (2001), vol.9, pp.205-218 et références citées) ou de polymères inertes biologiquement (Maas C.S. et col., Facial Plast. Surg. Clin. North Am. (2001), vol.9, pp.219-227 et références citées). Il est aussi avantageusement proposé au niveau cutané des produits à base de toxine botulique.

En ce qui concerne les problèmes affectant l'oeil et sa cornée, ils sont souvent corrigés avec le recours à des techniques chirurgicales, telles le laser ou la kératinoplastie, ou encore par le port de lentilles de contact.

D'une façon générale, quel que soit le type de tissu conjonctif, les techniques existantes de comblement ou de cicatrisation ne font jamais état d'une action s'exprimant primairement et favorablement sur l'organisation extra-cellulaire du réseau glycoprotéinique sur lequel repose l'architecture générale des tissus conjonctifs, en d'autres termes d'un quelconque effet visant une (ré)organisation non physiologique de l'architecture des constituants protéiniques de la matrice extra-cellulaire des tissus conjonctifs lésés.

En outre, pour les substances injectables d'origine naturelle, en plus des classiques problèmes d'allergie, un autre désavantage quant à leur utilisation, particulièrement en dermatologie cosmétique, est leur temps d'activité limité puisque dépendant de leur catabolisme tissulaire. En effet, leur cinétique d'effet montre un fort effet de comblement "post-injection" des volumes dépressionnaires, peu durable toutefois dans le temps et justifiant la nécessité de renouveler rapidement le traitement.

Il a maintenant été découvert de manière inattendue, et c'est le fondement de l'invention, que certains composés organo-siliciés, introduits *in situ* en concentrations supra physiologiques dans du derme lésé, permettent l'obtention, sans action pharmacologique directe, d'une organisation structurelle des fibres protéiniques et glycoprotéiniques de la matrice extra-cellulaire, ce qui permet de remédier aux inconvénients mentionnés ci-dessus.

Cette organisation non naturelle et contrainte est favorable au maintien ou au rétablissement de la matrice extra-cellulaire ; elle représente en effet un état d'arrangement structurel tissulaire non physiologique, intermédiaire, qui permet ultérieurement le recouvrement de la structure native du derme lésé.

Tout tissu conjonctif, tel que le derme, est en effet caractérisé par une matrice extra-cellulaire organisée tridimensionnellement par l'agencement de fibres protéiniques et glycoprotéiniques sur lesquelles sont fixés les composants cellulaires.

Une caractéristique majeure et récurrente des dégradations ou altérations afférentes à tout tissu conjonctif lésé est une modification et une perte systématiques de l'architecture de celui-ci, observées au niveau de l'organisation matricielle protéinique du compartiment extra-cellulaire, et généralement associées à une perte de volume dudit tissu. Il est alors fréquemment remarqué une épaisseur réduite, avec rigidification ou non, de ce dernier.

En conséquence, et compte tenu de la structure physiologique d'un tissu conjonctif et de sa matrice extra-cellulaire parfaitement organisée dans les trois dimensions, il est aujourd'hui perçu tout l'intérêt de maintenir une telle structure ou de la rétablir si elle est altérée suite à l'effet du processus chronologique de vieillissement ou à l'action d'un traumatisme quelconque, ou en réponse aux effets adverses de divers traitements correctifs. Ledit rétablissement constitue alors une étape indispensable au recouvrement ultérieur de la fonction physiologique originelle du tissu conjonctif lésé.

L'invention a donc été développée dans ce contexte.

Ainsi, selon un premier aspect, l'invention a pour objet l'utilisation d'au moins un composé organo-silicié de formule générale (I) suivante :

RₓSi(OH)₄₋ₓ (I)

dans laquelle :
R est un (C₁-C₄)alkyle, un (C₂-C₅)alcényle ou un aryle,
x = 1 à 3,
le(s)dit(s) composé(s) organo-silicié(s) étant stabilisé(s) par un acide carboxylique, un glycosaminoglycane, ou un sel de ces composés,
pour la fabrication d'une solution injectable destinée au traitement par administration in situ du derme lésé par contrainte de l'organisation des fibres protéiniques et glycoprotéiniques de la matrice extra-cellulaire du derme, ladite solution injectable contenant une dose supraphysiologique dudit (desdits) composé(s), comprise entre 0,1 et 50 mg et de préférence entre 1 et 25 mg par cm³ de derme traité et par solution administrée.

Par "derme lésé" on entend selon la présente invention du derme dégradé ou altéré *in vivo.* Ces dégradations ou altérations *in vivo* peuvent résulter aussi bien du processus naturel de vieillissement et de ses différents facteurs intrinsèques ou extrinsèques (chronologiques, génétiques, actiniques, comportementaux, endocriniens, cataboliques et mécaniques) que de processus pathologiques (maladie, blessure) ou après l'intervention d'un traitement "correctif" (chirurgie plastique, laser, traitements médicamenteux iatrogènes, opothérapie, lentilles de contact, etc.). Dans ces derniers cas, les altérations observées sont en général moins marquées et sont la conséquence des effets adverses des traitements correctifs.

Dans la formule (I), on entend par groupe alkyle en C₁-C₄ une chaîne hydrocarbonée ayant de 1 à 4 atomes de carbone, linéaire, ramifiée, ou bien encore cyclique. Un tel groupe est notamment un groupe méthyle, éthyle, propyle, butyle, 1-méthyléthyle, 1-méthylpropyle, 2-méthylpropyle, 1,1-diméthyléthyle et cyclopropylméthyle.

Par aryle, on entend un noyau phényle, un noyau 1- ou 2-naphtyle ou un noyau 2- ou 3-thiényle.

Par groupe alcényle en C₂-C₅, il faut comprendre un groupe comprenant 2 à 5 atomes de carbone dont 2 consécutifs sont liés par une liaison éthylénique, par exemple un groupe -CH₂-CH=CH-CH₂-, -CH=CH-CH₂-, -CH=C(CH₃)-CH₂-, CH₂-CH=CH- ou -CH=CH-CH(CH₃)-.

Comme exemple d'acide carboxylique, on peut citer notamment l'acide salicylique, l'acide glutamique, l'acide lactique, l'acide pyrrolidinone carboxylique, l'acide niflurique et/ou leurs sels.

Comme exemple de glycosaminoglycane, on peut citer notamment l'acide hyaluronique, le sulfate de chondrditine, le sulfate de kératane.

Les composés organo-siliciés sont de préférence stabilisés par l'acide salicylique, l'acide hyaluronique ou un sel de ceux-ci.

Des composés organo-siliciés particulièrement préférés sont choisis parmi le monométhylsilanetriol ou le diméthylsilanediol, tout particulièrement le monométhylsilanetriol.

De manière particulièrement préférée, on utilise selon 1'"invention du salicylate de monométhylsilanetriol.

Les composés organo-siliciés selon l'invention se présentent sous la forme de monomères, d'oligomères, ou d'un mélange de monomères et d'oligomères.

L'état en solution des composés organo-siliciés de l'invention peut être représenté par la formule (II) suivante :

[(T0)ᵣ + (T1)ₛ + (T2)ₜ] (II)

dans laquelle :
T0, T1 et T2 ont respectivement les formules : 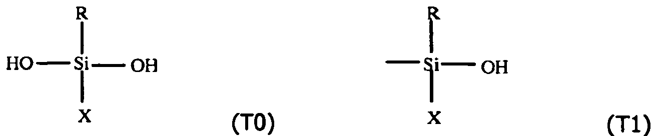 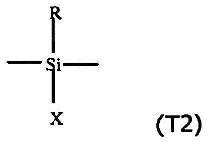 où :
   R est tel que défini ci-dessus pour les composés (I),
   X représente un groupe hydroxyle ou un radical R tel que défini ci-dessus, et
   r, s et t sont tels que 1 ≤ r+s+t ≤ 8.

Le procédé de préparation des composés organo-siliciés selon l'invention est décrit notamment dans les demandes de brevet publiées sous les numéros FR 2 158 068, FR 2 610 522 et EP 0 289 366.

Les composés de l'invention permettent un apport supraphysiologique de silicium exogène, apport important puisqu'il est établi qu'une diminution de la teneur en silicium naturel dans l'organisme, notamment avec l'âge, entraîne une destructuration des tissus conjonctifs (Charnot Y. et al., Lyon Med. (1971), vol.226, pp.85-88).

Les composés de l'invention sont par ailleurs des produits de synthèse, chimiquement stables, et par conséquent non métabolisables. Ils sont sans risque d'allergies ni d'effets secondaires et sont dotés d'une excellente hydrosolubilité, ainsi que d'une tolérance et innocuité démontrées.

Les composés selon la présente invention, introduits localement en doses supraphysiologiques dans du derme lésé, permettent de contraindre l'organisation des complexes protéine/glycosaminoglycane assurant l'intégrité structurelle du derme, via des liaisons chimiques siloxanes avec les fibres protéiniques et glycoprotéiniques de la matrice extra-cellulaire. Il en résulte une organisation non naturelle (ou supraphysiologique) du réseau fibrillaire extra-cellulaire très favorable à la régénération dudit derme, cette réorganisation "silanol-induite" conditionnant alors l'activité biologique globale du derme. Il est cependant à souligner que l'effet recherché n'est pas obtenu de façon dose-dépendante et qu'il se distingue donc clairement d'une action pharmacologique directe, en conséquence d'un traitement médicamenteux.

Les composés organo-siliciés selon l'invention permettent donc une organisation supra-moléculaire non physiologique des fibres constitutives du réseau protéinique extra-cellulaire du derme altéré, la cinétique afférente s'oppose diamétralement à celle observée avec des produits standards de comblement. Selon l'invention, elle est effectivement la suivante : sans effet de comblement jusqu'à une heure après l'injection ou le traitement, l'action des composés organo-siliciés sur le réseau fibrillaire extra-cellulaire se manifeste alors, progressivement et corrélativement à l'organisation supra-moléculaire induite. Le volume quasi-natif est en conséquence ainsi récupéré quelques jours après l'injection, avec une "récupération" stable dans le temps.

La demanderesse a en effet montré que les résultats obtenus avec les composés organo-siliciés de l'invention sont stables dans le temps, puisque maintenus sur une période d'un an, indépendants d'un quelconque métabolisme physiologique "post-introductif".

Comme le montre les tests ci-après, les effets recherchés sont avant tout de nature physique avec une amélioration des propriétés mécaniques du derme. Des effets pharmacologiques, secondaires au recouvrement d'une architecture tissulaire fonctionnelle, indirects et multiples pourront être toutefois aussi observés, leur nature hétérogène et multiaxiale justifiant, d'ailleurs, l'impossibilité d'un effet inducteur pharmacologique primaire et direct. En outre, en aucune manière, ces effets ne peuvent prédominer sur ceux résultant de la contrainte physique exercée sur l'architecture du derme altéré par les composés de l'invention.

Les composés organo-siliciés selon l'invention sont introduits localement dans du derme lésé à des doses supraphysiologiques afin de modifier physiquement et de façon non naturelle l'architecture dudit derme.

D'un point de vue chimique, on aurait pu penser qu'une introduction locale de ces doses supraphysiologiques de composés organo-siliciés exogènes entraînerait la formation de polymères de type silicone, inertes et en conséquence inefficaces. Ce n'est absolument pas ce qui est observé dans la présente invention.

A ces doses supraphysiologiques, on aurait également pu craindre que la contrainte non physiologique exercée sur l'organisation des protéines de la matrice extra-cellulaire conduise de ce fait à un état d'arrangement structurel toxique préjudiciable pour la zone de derme traité ne permettant pas l'effet recherché. Il n'en est rien avec les composés organo-siliciés de l'invention.

Les composés organo-siliciés selon l'invention sont administrés sous la forme d'une solution injectable. Ces préparations pharmaceutiques peuvent être préparées selon les techniques couramment utilisées en galénique.

Une telle préparation pharmaceutique comprend le composé organo-silicié stabilisé en une quantité exprimée en poids allant de 0,1% environ à 5% environ, en particulier de 0,1% environ à 1% environ, et de préférence égale à 0,5% environ.

Les composés organo-siliciés selon l'invention peuvent également être appliqués *ex vivo* sur un explant cutané prélevé d'une zone donneuse saine et qui est ensuite réimplanté, après croissance, sur une peau altérée.

Les altérations d'organisation de la matrice extracellulaire susceptibles d'être traitées par les composés de l'invention sont des altérations dermiques, qui comprennent les dépressions induites par le vieillissement ou après l'intervention d'un traitement correctif, ou d'origine traumatique, tout particulièrement les rides, les ridules, les cicatrices, les vergétures, et les escarres. Préférentiellement, le mode d'administration locale pour ce type d'altérations est les injections intradermiques.

Les composés organo-siliciés selon l'invention peuvent être utilisés seuls ou en association avec un dispositif médical (au sens de la Directive Communautaire 93/42/CEE) biocompatible choisi parmi le groupe des dispositifs médicaux implantables, de préférence les prothèses, les ciments osseux, les lentilles de courbure inverse et les dispositifs implantables destinés à la délivrance de médicaments ; des produits et sutures résorbables ; des produits viscoélastiques ; des produits hémostatiques ; des matrices bioacceptables, de préférence les microéponges ; des armatures intra-artérielles, de préférence les stents ; de tout matériel médical stérile en contact avec le patient, de préférence les compresses et les pansements ; des dispositifs utilisés dans l'art dentaire, de préférence les produits de comblement dentaire ou amalgames, les prothèses et les couronnes.

Lorsque le dispositif médical est de nature à être utilisé sur une plaie ouverte (cas typique d'une compresse ou d'un pansement) il est possible de le traiter au préalable avec une préparation comprenant les composés organo-siliciés de l'invention.

Les composés organo-siliciés selon l'invention peuvent être également utilisés en association avec un agent pharmacologique ne permettant pas une contrainte telle que précédemment décrite sur l'organisation de la matrice extracellulaire. Un tel agent est choisi parmi les anti-inflammatoires, les bactériostatiques et les bactéricides, c'est-à-dire des agents capables de conserver le tissu conjonctif lésé dans un état convenable pour le traitement visé.

Selon un autre aspect, invention concerne des composés organo-siliciés tels que définis aux revendications 12 et 13.

L'invention sera mieux comprise à l'aide des tests décrits ci-après, donnés à titre purement illustratif. Dans ces tests, on a utilisé les préparations suivantes, dans lesquelles les quantités sont exprimées en pourcentage en poids :

### Préparation 1 : solution injectable, isotonique, pH 5,0 à 6,0

* salicylate de monométhylsilanetriol : 0,5 %
* chlorure de sodium : 0,45 %
* eau pour préparation injectable qsp : 5 ml

### Préparation 2 : crème

* hyaluronate de monométhylsilanetriol : 0,5 %
* parrafine : 2%
* immidazolidinyl urée : 0,3 %
* sodium méthyl-paraben : 0,10 %
* propylparaben 0,05 %
* glycérine ou équivalent :13 %
* alcool cétylstéarylique et éther cétostéarylique : 10 %
* parrafine liquide : 5 %,
* édétate de sodium : 0,01 %
* acide ascorbique (facultatif) : 1 %
* eau purifiée qsp : 100 %

Dans les tests, on a mesuré les activités collagénase, élastase et glucose-6-phosphate déshydrogénase selon les protocoles décrits ci-après :

### Activité collagénase

Une solution à 0,5 mg /ml de PZ-L Pro-L Leu-L Gly-L Pro-D Arg a été réalisée dans un tampon Tris-HCl (25 mM, pH 7,0 ; 2,5 mM de CaCl₂). 0,5 ml de la solution de substrat ont été incubés à 37°C avec 200 µl d'extrait tissulaire pendant 2 h 30. A différents temps, 100 µl du milieu réactionnel ont été prélevés et ajoutés à 250 µl d'acide citrique à 0,5% (arrêt de la réaction) puis à 1,25 ml d'acétate d'éthyle. L'augmentation de densité optique (mD.O./min) et corrélée à la protéolyse du substrat a été suivie à 320 nm.

### Activité élastase

Du N-succinyl-Ala-Ala-Pro-Leu p-nitroanilide en solution dans un tampon Tris-HCl (10mM pH 8) à 0,5 g/l à été incubé à 37°C avec 0,2 ml d'extrait tissulaire dans un volume final de 1,2 ml pendant 5 heures. L'augmentation de densité optique (mD.O./min) et corrélée à la protéolyse du substrat a été suivie à 320 nm.

### Activité glucose-6-phosphate déshydrogénase

0,8 ml d'extrait tissulaire ont été incubés avec 20 µl de solution à 2,4 mM de NADP et à 24,4 mM de glucose 6-phosphate (G6P) en tampon phosphate (0,1M pH 7,4). L'augmentation de densité optique (mD.O./min) et corrélée à la réduction du substrat a été suivie à 340 nm à 25°C.

### Test 1 : Effet du salicylate de monométhylsilanetriol et du hyaluronate de monométhylsilanetriol sur l'organisation matricielle dermique (comparaison avec un témoin). Administration par injections intradermiques ou à l'aide d'une crème standard

L'action quantitative et qualitative des deux composés organo-siliciés sur des rats Hairless et les altérations d'organisation de leur matrice extra-cellulaire (MEC) cutanée a été évaluée, et de manière régiosélective. En effet, un volume dermique de 0,5 cm³ environ a été prélevé puis remplacé chirurgicalement par un dispositif médical implantable, que sont les microéponges en polyvinylalcool. Ces dernières sont usuellement utilisées pour la transplantation de cellules (Mooney D.J., J. Biomed. Mat Res(1995), vol.29, pp.959) car biocompatibles, de haute porosité et capables d'absorber 98% de leur volume.

La re-colonisation de l'implant (ou dispositif médical implantable) par le tissu dermique est alors observée sous l'effet des composés organo-siliciés. Le premier, sous la forme d'une solution injectable à 0,5% en poids de salicylate de monométhylsilanetriol (SalSi-30H ; préparation 1, (0,2 ml)), est injecté intradermiquement, le second, sous la forme d'une crème standard à 0,5% en poids de hyaluronate de monométhylsilanetriol (HyalSi-3OH ; préparation 2, (100 mg)), est appliqué topiquement. Les composés organo-siliciés sont administrés à raison d'une fois par jour pendant 2 mois.

L'évaluation quantitative a été réalisée via une mesure des cinétiques des marqueurs d'activité tissulaire dans la zone implantée. Les marqueurs suivis ont été des marqueurs biologiques témoignant de l'état du tissu, à savoir les enzymes "collagénase" et "élastase" qui rendent compte, avec leurs activités, du turn-over des protéines de la matrice extracellulaire, et l'enzyme "G6PDH" dont l'activité est un marqueur du niveau énergétique des cellules (équilibre redox cellulaire). L'évaluation qualitative a, elle, été réalisée avec le prélèvement chirurgical d'une partie de cet implant à différent temps de l'expérimentation. Après fixation, des coupes de 5 µm et une coloration H.E ou Trichrome-Masson ont été réalisées, ainsi qu'une analyse histologique de l'organisation du collagène de la MEC.

Les résultats sont présentés dans les Tableaux 1 et 2.

**Tableau 1 : évaluation quantitative**

| | | Semaine post-implantation | | | | | |
|---|---|---|---|---|---|---|---|
| | | 1 | 2 | 3 | 4 | 5 | 8 |
| Activité collagénase* | **Témoin** | 3,1±0,3 | 5,6±0,4 | 10,5±1,3 | 12,9±0,9 | 6,7±1,4 | 4,2±0,6 |
| | **Témoin + SalSi-3OH** | 3,6±0,5 | 6,1±0,8 | 13±1,7 | 11,2±0,8 | 8,1±1,3 | 3,9±0,5 |
| | **Témoin + HyalSi-3OH** | 3,4±0,3 | 5,9±0,7 | 12,0±1,3 | 14,1±3,1 | 7,3±1,6 | 3,5±1,4 |
| Activité élastase* | **Témoin** | 2,8±0,4 | 6,4±0,8 | 8,2±1,0 | 7,4±1,5 | 4,3±0,7 | 2,2±0,4 |
| | **Témoin + SalSi-3OH** | 3,0±0,6 | 6,8±1,3 | 9,5±1,3 | 8,4±1,6 | 5,1±0,9 | 2,0±0,3 |
| | **Témoin + HyalSi-3OH** | 3,4±0,5 | 7,8±2,2 | 10,5±2,9 | 9,4±2,4 | 5,6±1,1 | 2,4±0,9 |
| Activité G6DPH* | **Témoin** | 6,4±1,2 | 11,5±1,4 | 22,7±4,1 | 39,5±3,8 | 30,3±5,2 | 5,9±0,8 |
| | **Témoin + SalSi-3OH** | 7,6±1,4 | 9,3±1,1 | 18,4±2,9 | 43,7±5,5 | 36,5±4,8 | 9,0±0,45 |
| | **Témoin + HyalSi-3OH** | 8,2±1,9 | 13,8±1,5 | 25,1±4,6 | 46,8±7,6 | 37,5±6,2 | 7,3±2,4 |

| | | | | | | | |
|---|---|---|---|---|---|---|---|
| * mD.O./min/mg de prot. | | | | | | | |

**Tableau 2 : évaluation qualitative**

| Semaine post-implantation | Aspect implant + analyse histologique **Témoin** | Aspect implant + analyse histologique **Témoin + SalSi-3OH** | Aspect implant + analyse histologique **Témoin + HyalSi-3OH** |
|---|---|---|---|
| 1 | début colonisation cellulaire | début colonisation cellulaire | début colonisation cellulaire |
| 2 | colonisation cellulaire (70à 90%) + début formation MEC | colonisation cellulaire (70à 90%) + début formation MEC | colonisation cellulaire (70 à 90%) + début formation MEC |
| 3 | formation MEC avec accumulation collagène non organisé | formation MEC avec présence collagène non organisé | formation MEC avec accumulation collagène non organisé |
| 4 | formation MEC avec accumulation collagène non organisé dans 30% des espaces de l'implant | formation MEC avec présence collagène non organisé dans 20% des espaces de l'implant + *collagène organisé dans 20% du l'implant* | formation MEC avec présence collagène non organisé dans 30% des espaces de l'implant + *collagène organisé dans 10% de l'implant* |
| 5 | formation MEC avec accumulation collagène non organisé dans l'ensemble de l'implant | formation MEC avec présence collagène non organisédans l'ensemble de l'implant + *collagène organisé dans 50% de l'implant* | formation MEC avec présence collagène non organisé dans l'ensemble de l'implant + *collagéne organisé dans 30% de l'implant* |
| 8 | formation MEC dans l'ensemble de l'implant + collagène organisé avec espace intrafibrillaire sur 10-20% de l'implant | formation MEC dans l'ensemble de l'implant + *collagène organisé dans 70% de l'implant* | formation MEC dans l'ensemble de l'implant + *collagène organisé dans 45% de l'implant* |

En présence des composés de l'invention, les marqueurs biologiques de la re-colonisation ont été exprimés normalement, caractérisant de ce fait l'absence de tout effet pharmacologique primaire et direct.

### Test 2 : Effet du salicylate de monométhylsilanetriol sur le comblement des rides et des dépressions cutanées (comparaison avec un témoin). Administration par injections intra-dermiques répétées in situ

Le salicylate de monométhylsilanetriol (SalSi-3OH ; préparation 1) a été administré par injections intradermiques au niveau des rides "patte d'oie" et/ou intersourcilières de 103 femmes (bilans paracliniques normaux), à raison de 3 séances d'injection espacées chacune d'une semaine (J0, S1 et S2).

Des visites de contrôle ont ensuite été réalisées à S6, S12, S24, S36 et S52, avec les effectifs suivants suite à quelques perdus de vue ou exclusion :
- de J0 à S12, 98 femmes d'âge moyen 49,5+/- 6 ans justifiant d'une correction au niveau des pattes d'oie (n=97) et/ou dans la région intersourcilière (n=89) ;
- de S24 à S52, 96 sujets dont 95 ont reçu les injections au niveau des pattes d'oie et 88 au niveau intersourcillier.

L'efficacité du salicylate de monométhylsilanetriol a été évaluée sur une année (52 semaines) et sur un volume de 0,5 cm³ environ à partir des techniques non invasives suivantes : iconographie macrophotographique, traitement informatique d'images sur empreintes cutanées des zones traitées, échelle d'évaluation visuelle analogique de 100mm par les volontaires lors de chaque visite.

La résorption du produit est complète une heure après l'injection, sans effet de comblement immédiatement après l'injection. Les volumes injectés de SalSi-3OH à J0, S1 et S2 ont été :

| | Pattes-d'oie (n=97) | Rides intersourcilières (n=89) |
|---|---|---|
| J0 | 0,38 ± 0,16 ml | 0,40 ± 0,15 ml |
| S1 | 0,32 ± 0,12 ml | 0,34 ± 0,11 ml |
| S2 | 0,26 ± 0,09 ml | 0,34 ± 0,13 ml |

Caractéristique d'une manifestation macroscopique d'une récupération du volume par recouvrement d'une organisation favorable du réseau glycoprotéique, le volume dépressionnaire à combler par le dispositif injectable diminue de J0 à S2.

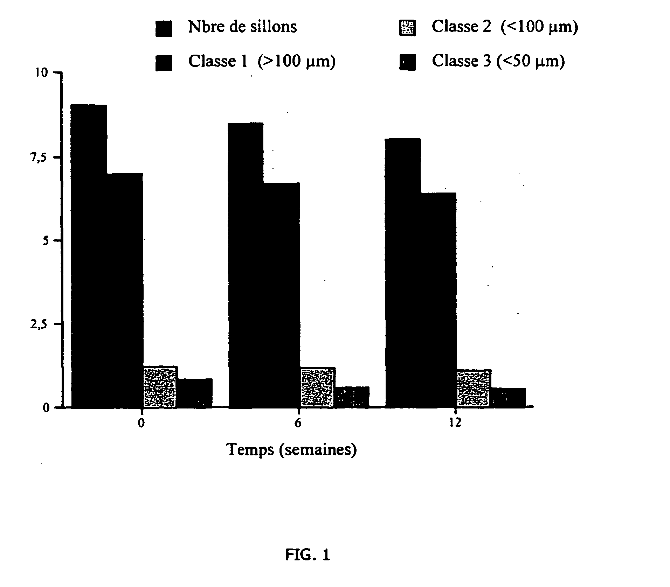

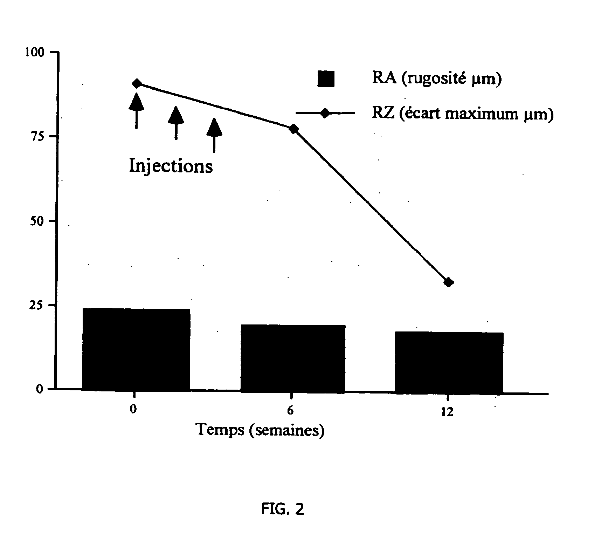

L'analyse du microrelief sur les rides siégeant en peau épaisse est représentée figures 1 et 2. L'observation d'une amélioration statistique significative a nécessité entre 6 et 12 semaines.

Les données obtenues à partir de l'échelle visuelle analogique ont fait l'objet d'une analyse de variance en mesures répétées sur le temps, suivie d'une analyse des contrastes. Les résultats sont présentés dans le Tableau 3 (l'unité utilisée est le µm).

**Tableau 3**

| | **Front** | **Pattes-d'oie** |
|---|---|---|
| **S1** | 18,67 ± 20,98 | 19,20 ± 19,14 |
| **S2** | 32,25 ± 23,23 | 31,23 ± 21,66 |
| **S6** | 35,33 ± 26,99 | 38,92 ± 25,97 |
| **S12** | 38,65 ± 25,85 | 41,63 ± 26,70 |
| **S24** | 39,55 ± 25,33 | 39,58 ± 24,89 |
| **S36** | 36,57 ± 24,55 | 39,52 ± 25,22 |
| **S52** | 34,58 ± 25,54 | 38,61 ± 26,10 |

Statistiquement, l'amélioration du relief cutané atteint en moyenne 40% après 3 mois sur tous les sites injectés, se maintient à 6 mois, et régresse très légèrement à 12 mois.

Toutefois significative sur tous les sites un an après la première injection, cette amélioration est le reflet d'une réorganisation et d'un recouvrement partiel du volume initial du derme en accord avec le mode d'activité décrit.

## Revendications

1. Utilisation d'au moins un composé organo-silicié de formule générale (I) suivante :
RₓSi(OH)₄₋ₓ (I)
dans laquelle :
R est un (C₁-C₄)alkyle, un (C₂-C₅)alcényle ou un aryle,
x = 1 à 3,
le(s)dit(s) compos(é) organo-silicié(s) étant stabilisé(s) par un acide carboxylique, un glycosaminoglycane, ou un sel de ces composés,
pour la fabrication d'une solution injectable destinée au traitement *in situ* du derme lésé par contrainte de l'organisation des fibres protéiniques et glycoprotéiniques de la matrice extra-cellulaire du derme, ladite solution injectable contenant une dose supraphysiologique dudit (desdits) composé(s), comprise entre 0,1 et 50 mg et de préférence entre 1 et 25 mg par cm³ de derme traité et par solution administrée.

2. Utilisation selon la revendication 1, dans laquelle l'acide carboxylique est choisi parmi l'acide salicylique, l'acide glutamique, l'acide lactique, l'acide pyrrolidinone carboxylique et l'acide niflumique.

3. Utilisation selon la revendication 1, dans laquelle le glycosaminoglycane est choisi parmi l'acide hyaluronique, le sulfate de chondroïtine et le sulfate de kératane.

4. Utilisation selon la revendication 1, dans laquelle le composé organo-silicié est stabilisé par l'acide salicylique, l'acide hyaluronique ou un sel de ceux-ci.

5. Utilisation selon l'une des revendications 1 à 4, dans laquelle le composé organo-silicié est le monométhylsilanetriol ou le diméthylsilanediol.

6. Utilisation selon la revendication 1, dans laquelle le composé de formule (I) stabilisé est le salicylate de monométhylsilanetriol.

7. Utilisation selon l'une des revendications 1 à 6, dans laquelle le composé organo-silicié stabilisé est présent dans la solution injectable, en une quantité exprimée en poids allant de 0,1% environ à 5% environ, en particulier de 0,1% environ à 1% environ.

8. Utilisation selon la revendication 7, dans laquelle le composé organo-silicié stabilisé est présent dans la solution injectable en une quantité exprimée en poids de 0,5% environ.

9. Utilisation selon l'une des revendications 1 à 8, dans laquelle la solution injectable permet de maintenir ou rétablir la structure des fibres protéiniques et glycoprotéiniques de la matrice extracellulaire du derme lésé.

10. Utilisation selon l'une des revendications 1 à 9, dans laquelle le(s) composé(s) organo-siliciés est (sont) utilisé(s) en association avec un dispositif médical choisi parmi les dispositifs médicaux implantables, les produits et sutures résorbables, les produits viscoélastiques, les produits hémostatiques, les matrices bioacceptables, les armatures intra-artérielles, les matériaux stériles et les dispositifs utilisés dans l'art dentaire.

11. Utilisation selon l'une des revendications 1 à 9, dans laquelle le(s) composé(s) organo-silicié(s) est(sont) utilisé(s) en association avec un agent pharmacologique choisi parmi les anti-inflammatoires, les bactériostatiques et les bactéricides.

12. Composé organo-silicié de formule générale (I) suivante :
RₓSi(OH)₄₋ₓ (I)
dans laquelle :
R est un (C₁-C₄)alkyle, un (C₂-C₅)alcényle ou un aryle,
x = 1 à 3,
ledit composé organo-silicié étant stabilisé par un acide carboxylique, un glycosaminoglycane, ou un sel de ces composés,
pour son utilisation dans le traitement du derme lésé, par administration *in situ* d'une solution injectable contenant une dose supraphysiologique dudit composé, comprise entre 0,1 et 50 mg et de préférence entre 1 et 25 mg par cm³ de derme traité et par solution administrée.

13. Composé organo-silicié stabilisé pour l'utilisation selon la revendication 12, qui est le salicylate de monométhylsilanetriol.

## Claims

1. Use of at least one organo-silicon compound of the following general formula (I):
RₓSi(OH)₄₋ₓ (I)
wherein:
R is a (C₁-C₄)alkyl, (C₂-C₅)alkenyl or aryl,
x = 1 to 3,
the said organo-silicon compound(s) being stabilized by a carboxylic acid, a glycosaminoglycan, or a salt of these compounds,
for the manufacture of an injectable solution intended for the *in situ* treatment of damaged dermis by constraining the organization of protein and glycoprotein fibres of the extra-cellular matrix of dermis, the said injectable solution containing a supra-physiological dose of the said compound(s), comprised between 0.1 and 50 mg, and preferably between 1 and 25 mg, per cm³ of treated dermis and by administered solution.

2. The use according to claim 1, wherein the carboxylic acid is chosen among salicylic acid, glutamic acid, lactic acid, pyrrolidinone carboxylic acid and niflumic acid.

3. The use according to claim 1, wherein the glycosaminoglycan is chosen among hyaluronic acid, chondroitin sulfate and keratan sulfate.

4. The use according to claim 1, wherein the organo-silicon compound is stabilized by salicylic acid, hyaluronic acid, or a salt thereof.

5. The use according to one of claims 1 to 4, wherein the organo-silicon compound is the monomethylsilanetriol or dimethylsilanediol.

6. The use according to claim 1, wherein the stabilized compound of formula (I) is monomethylsilanetriol salicylate.

7. The use according to one of claims 1 to 6, wherein the stabilized organo-silicon compound is present in the injectable solution in an amount expressed in weight from about 0.1% to about 5%, in particular from about 0.1% to about 1%.

8. The use according to claim 7, wherein the stabilized organo-silicon compound is present in the injectable solution in an amount expressed in weight of about 0.5%.

9. The use according to one of claims 1 to 8, wherein the injectable solution allows the maintenance or recovery of the structure of protein and glycoprotein fibres of the extra-cellular matrix of the damaged dermis.

10. The use according to one of claims 1 to 9, wherein the organo-silicon compound(s) is (are) used in combination with a medical device chosen among implantable medical devices, resorbable products and sutures, viscoelastic products, haemostatic products, bio-acceptable matrices, intra-arterial armatures, sterile medical devices and devices used in dentistry.

11. The use according to one of claims 1 to 9, wherein the organo-silicon compound(s) is (are) used in combination with a pharmacological agent chosen among anti-inflammatory drugs, bacteriostatics, and bactericides.

12. An organo-silicon compound of general formula (I):
RₓSi(OH)₄₋ₓ (I)
wherein:
R is a (C₁-C₄)alkyl, (C₂-C₅)alkenyl or aryl,
x = 1 to 3,
the said organo-silicon compound(s) being stabilized by a carboxylic acid, a glycosaminoglycan, or a salt of these compounds,
for use in the treatment of damaged dermis by *in situ* administration of an injectable solution containing a supra-physiological dose of said compound, comprised between 0.1 and 50 mg, and preferably between 1 and 25 mg, per cm³ of treated dermis and by administered solution.

13. Stabilized organo-silicon compound for the use according to claim 12, which is monomethylsilanetriol salicylate.

## Patentansprüche

1. Verwendung wenigstens einer organischen Siliziumverbindung der folgenden allgemeinen Formel (I):
RₓSi(OH)₄₋ₓ (I)
worin:
R ein (C₁-C₄)Alkyl, ein (C₂-C₅)Alkenyl oder ein Aryl ist,
x = 1 bis 3,
wobei die organische(n) Siliziumverbindung(en) durch eine Carbonsäure, ein Glykosaminoglykan oder ein Salz dieser Verbindungen stabilisiert ist (sind),
für die Herstellung einer injizierbaren Lösung, die bestimmt ist für die In-situ-Behandlung der geschädigten Dermis durch Erzwingen der Organisation der Protein- und Glycoproteinfasern der extrazellulären Matrix der Dermis, wobei die injizierbare Lösung eine supraphysiologische Dosis der Verbindung(en) zwischen 0,1 und 50 mg und vorzugsweise zwischen 1 und 25 mg pro cm³ behandelter Dermis und pro verabreichter Lösung enthält.

2. Verwendung nach Anspruch 1, wobei die Carbonsäure aus Salicylsäure, Glutaminsäure, Milchsäure, Pyrrolidinon-Carbonsäure und Nifluminsäure ausgewählt ist.

3. Verwendung nach Anspruch 1, wobei das Glykosaminoglykan aus Hyaluronsäure, Chondroitinsulfat und Keratansulfat ausgewählt ist.

4. Verwendung nach Anspruch 1, wobei die organische Siliziumverbindung durch Salicylsäure, Hyaluronsäure oder ein Salz dieser stabilisiert ist.

5. Verwendung nach einem der Ansprüche 1 bis 4, wobei die organische Siliziumverbindung Monomethylsilantriol oder Dimethylsilandiol ist.

6. Verwendung nach Anspruch 1, wobei die stabilisierte Verbindung der Formel (I) Monomethylsilantriol-Salicylat ist.

7. Verwendung nach einem der Ansprüche 1 bis 6, wobei die stabilisierte organische Siliziumverbindung in der injizierbaren Lösung in einer in Gewicht ausgedrückten Menge von etwa 0,1 % bis etwa 5 %, insbesondere von etwa 0,1 % bis etwa 1 % vorhanden ist.

8. Verwendung nach Anspruch 7, wobei die stabilisierte organische Siliziumverbindung in der injizierbaren Lösung in einer in Gewicht ausgedrückten Menge von etwa 0,5 % vorhanden ist.

9. Verwendung nach einem der Ansprüche 1 bis 8, wobei die injizierbare Lösung ermöglicht, die Struktur der Protein- und Glycoproteinfasern der extrazellulären Matrix der geschädigten Dermis zu erhalten oder wiederherzustellen.

10. Verwendung nach einem der Ansprüche 1 bis 9, wobei die organische(n) Siliziumverbindung(en) in Verbindung mit einem Medizinprodukt verwendet wird (werden), das aus den implantierbaren Medizinprodukten, den resorbierbaren Produkten und Nahtmaterialien, den viskoelastischen Produkten, den hämostatischen Produkten, den biologisch verträglichen Matrizes, den intraarteriellen Verstärkungen, den sterilen Materialien und den in der Zahnheilkunde verwendeten Produkten ausgewählt ist.

11. Verwendung nach einem der Ansprüche 1 bis 9, wobei die organische(n) Siliziumverbindung(en) in Verbindung mit einem pharmakologischen Mittel verwendet wird (werden), das aus den Entzündungshemmern, den Bakteriostatika und den Bakteriziden ausgewählt ist.

12. Organische Siliziumverbindung der folgenden allgemeinen Formel (I):
RₓSi(OH)₄₋ₓ (I)
worin:
R ein (C₁-C₄)Alkyl, ein (C₂-C₅)Alkenyl oder ein Aryl ist,
x = 1 bis 3,
wobei die organische Siliziumverbindung durch eine Carbonsäure, ein Glykosaminoglykan oder ein Salz dieser Verbindungen stabilisiert ist,
für ihre Verwendung bei der Behandlung der geschädigten Dermis durch Insitu-Verabreichung einer injizierbaren Lösung, die eine supraphysiologische Dosis der Verbindung zwischen 0,1 und 50 mg und vorzugsweise zwischen 1 und 25 mg pro cm³ behandelter Dermis und pro verabreichter Lösung enthält.

13. Stabilisierte organische Siliziumverbindung für die Verwendung nach Anspruch 12, die Monomethylsilantriol-Salicylat ist.
